# EUROPÄISCHE PATENTSCHRIFT

(11) **EP 1 357 798 B1**
(45) Veröffentlichungstag und Bekanntmachung des Hinweises auf die Patenterteilung: **19.04.2006**
(21) Anmeldenummer: 02716028.2
(22) Anmeldetag: 05.02.2002
(51) Int. Cl.: A23B 9/02, A23L 3/16, A61K 8/00, A61L 2/06

(54) **BESCHICKUNGSSYSTEM FÜR EINE ANLAGE ZUM ENTKEIMEN VORZUGSWEISE VON LEBENSMITTELN, PHARMAZEUTISCHEN ODER KOSMETISCHEN PRODUKTEN**
FEEDING SYSTEM FOR AN INSTALLATION FOR STERILISATION, PREFERABLY OF FOOD, PHARMACEUTICAL PRODUCTS OR COSMETIC PRODUCTS
SYSTEME DE CHARGEMENT POUR UNE INSTALLATION DE STERILISATION, DE PREFERENCE D'ALIMENTS, DE PRODUITS PHARMACEUTIQUES OU DE PRODUITS COSMETIQUES

(30) Priorität: 05.02.2001 CH 195012001
(43) Veröffentlichungstag der Anmeldung: 05.11.2003
(73) Patentinhaber: STEAMLAB SYSTEMS AG NATURAL PASTEURIZATION, CH-4103 Bottmingen (CH)
(72) Erfinder: SACCAVINO, Cesare, CH-4147 Aesch (CH); KRAMER, Roland, CH-4146 Hochwald (CH); SACCAVINO, Mario, CH-4402 Frenkendorf (CH)
(74) Vertreter: Luchs, Willi
(86) Internationale Anmeldenummer: PCT/CH2002/000066
(87) Internationale Veröffentlichungsnummer: WO 2002/062151

(56) Entgegenhaltungen:
- EP-A- 0 998 855
- PATENT ABSTRACTS OF JAPAN vol. 1999, no. 01, 29. Januar 1999 (1999-01-29) -& JP 10 262573 A (HISAKA WORKS LTD), 6. Oktober 1998 (1998-10-06)

## Beschreibung

Die Erfindung betrifft ein Beschickungssystem für eine Anlage zum Entkeimen vorzugsweise von Lebensmitteln, pharmazeutischen oder kosmetischen Produkten gemäss dem Oberbegriff des Anspruches 1.

Aus der Druckschrift EP 0 998 855 A2 ist bereits ein System mit einem Beschickungsbehälter bekannt, bei dem das zu entkeimende Gut in das Innere von röhrenartigen, dampfdurchlässigen Hohlkörpern eingefüllt wird, die in eine Kammer mit Dampfanschluss eingesetzt sind. Mit dieser Anordnung wird das Entkeimungsgut durch diese schmalen Einheiten vollständig entkeimt. Nachteilig ist die relativ umständliche Beschickung und Entleerung dieser röhrenartigen Hohlkörper.

Die gattungsbildende JP-A-10262573 offenbart eine Anlage der eingangs genannten Art, mit einer von einem Beschickungsbehälter gebildeten Kammer, die ein oder mehrere dampfdurchlässige, röhrenartige Hohlkörper aufweist, wobei das Entkeimungsgut in die Kammer zwischen die Hohlkörper (bzw. um den einen Hohlkörper herum) eingefüllt wird. Bei diesem System ist es von Nachteil, dass der Dampfzutritt zu dem in die im Querschnitt quadratische Kammer eingefüllten Entkeimungsgut ungleichmässig ist.

Mit der vorliegenden Erfindung soll die Aufgabe gelöst werden, ein Beschickungssystem nach der eingangs erläuterten Gattung zu schaffen, bei dem der Dampfzutritt zum zu entkeimenden Gut verbessert wird. Ferner soll eine Veränderbarkeit der Materialschichten ermöglicht werden.

Diese Aufgabe ist erfindungsgemäss durch ein Beschickungssystem mit den Merkmalen des Anspruches 1 gelöst.

Mit dem erfindungsgemässen Beschickungssystem kann das Entkeimungsgut auf sehr rationelle und gleichsam optimale Weise entkeimt werden. Durch die Anordnung der plattenförmigen Hohlkörper in einer Reihe voneinander beabstandet entstehen grossflächige und gleichmässige Berührungsebenen zum Entkeimungsgut. Hieraus resultiert ein weitgehender Erhalt der Homogenität und eine gleichmässige Dampfdurchdringung der zu entkeimenden Produkte. Des weiteren können durch Herausnehmen zum Beispiel jedes zweiten Hohlkörpers verschiedene Materialien in unterschiedlich dicken Schichten homogen entkeimt werden.

In der Zeichnung ist ein Ausführungsbeispiel der Erfindung sowie weitere Vorteile derselben schematisch dargestellt. Es zeigen:
- Fig.1: eine Seitenansicht eines erfindungsgemässen Beschickungsbehälters mit plattenförmigen Hohlkörpern, wobei der Behälter geschnitten dargestellt ist,
- Fig.2: eine Draufsicht auf den Beschickungsbehälter nach Fig.1,
- Fig.3: einen Schnitt durch einen Beschickungsbehälter, und
- Fig.4: eine Draufsicht auf den Aufnahmebehälter mit vier rohrförmigen Hohlkörpern.

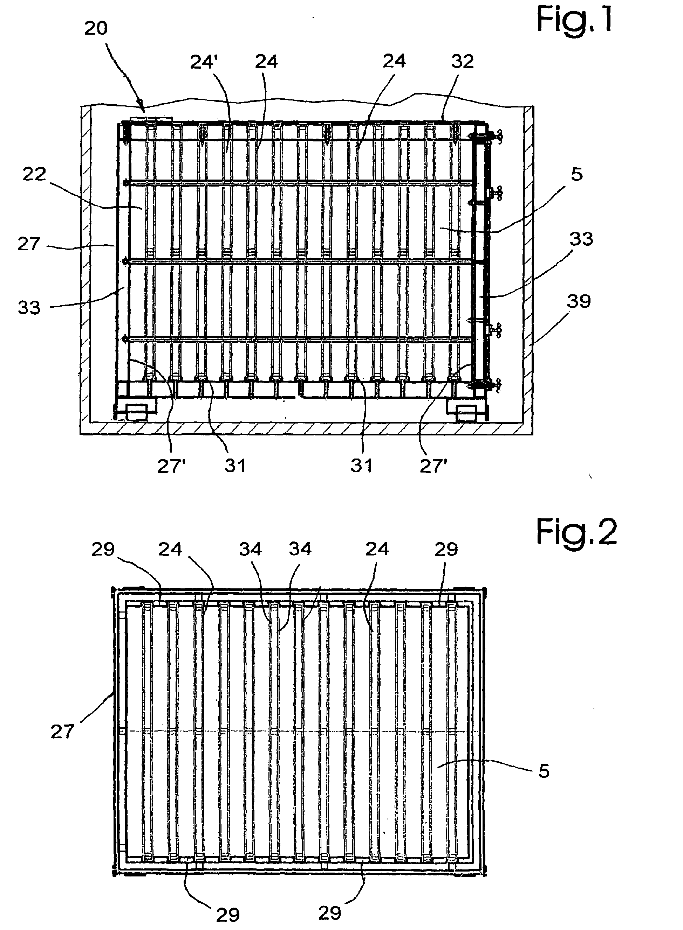

Ein Beschickungssystem gemäss Fig. 1 ist in einer Entkeimungsanlage 20 realisiert, die ein in einen angedeuteten Autoklaven 39 oder dergleichen stellbaren Beschickungsbehälter 27 mit einer innenseitigen Kammer 22 aufweist. Es können vorzugsweise mehrere solcher Behälter 27 über- und nebeneinander in den Autoklav 39 hineingestellt werden. In einer solchen Entkeimungsanlage werden vorzugsweise Lebensmittel, pharmazeutische oder kosmetische Produkte bzw. Rohstoffe hiervon nur an der Oberfläche jedes einzelnen Partikels entkeimt. Solche Lebensmittel können beispielsweise Pfefferkörner, Teeblätter, Pilze, Wurzeln oder Mehle oder andere sein.

Erfindungsgemäss sind in der Kammer 22 mehrere dampfdurchlässige, im Abstand zueinander angeordnete Hohlkörper 24 enthalten, zwischen diesen das Entkeimungsgut 5 einfüllbar ist, wobei diese Hohlkörper 24 eine Dampfdurchdringung ermöglichen.

In der Kammer 22 sind plattenförmige Hohlkörper 24 hineingestellt, die in einer Reihe beabstandet zueinander angeordnet sind, zwischen diesen das Entkeimungsgut 5 einfüllbar ist. Der in dem Autoklav 39 hineingestellte quaderförmige Behälter 27 hat auf seiner Oberseite Querstangen 32 oder dergleichen, mittels denen verhindert werden kann, dass die Hohlkörper 24 bei einem Abkippen des Behälters 27 herausfallen. Des weiteren ist der Behälter 27 an seinen Aussen- und Innenwänden 27' ebenfalls dampfdurchlässig ausgebildet, und es kann durch die doppelwandige dampfdurchlässige Behälterwand mit dem gebildeten Hohlraum 33 Dampf in die Innenseite eingeführt und das an der Innenseite anliegende Entkeimungsgut 5 entsprechend entkeimt werden. Die Wand kann zum Beispiel aus einem PP-Gewebe oder aber auch aus anderen dampfdurchlässigen Materialien, wie Stahlgewebe, Kunststoffe, Textilgewebe oder anderen bestehen.

Die plattenförmigen Hohlkörper 24 sind schubladenartig in Schienen 29 auf der Innenseite des Behälters 27 einschiebbar angeordnet. Damit lässt sich die Anzahl der eingesetzten Hohlkörper je nach Entkeimungsgut variieren. So kann beispielsweise bei einem grobkörnigeren Gut jeder zweite Hohlkörper weggelassen werden, indessen bei einem mehlartigen Gut sind mit Vorteil sämtliche Hohlkörper eingesetzt. Auf der Unterseite des Behälters 27 sind ebenfalls noch Führungsleisten 31 angeordnet, in denen die senkrecht angeordneten Hohlkörper 24 unten zusätzlich zentriert sind.

Zweckmässigerweise erstrecken sich die Hohlkörper 24 jeweils über den gesamten Querschnitt des Behälters 27, so dass zwischen diesen kein Spalt vorhanden ist. Sie bestehen jeweils aus einem plattenförmigen Kasten, der mit einem Hohlraum 24' versehen ist. Die vom Entkeimungsgut beaufschlagten Seitenwände 34 sind aus einem dampfdurchlässigen Material hergestellt, beispielsweise aus einem Gewebe, welches aus Kunststoff, Edelstahl, Textilien oder einer Kombination dieser Materialien bestehen.

Im Prinzip könnte das Behälter auch derart ausgestaltet sein, dass die plattenförmigen Hohlkörper auch horizontal oder schräg in den Behälter eingeschoben werden könnten. Die einzelnen Hohlkörper 22 könnten auch alle oder teilweise mit einem direkten Dampfanschluss versehen sein.

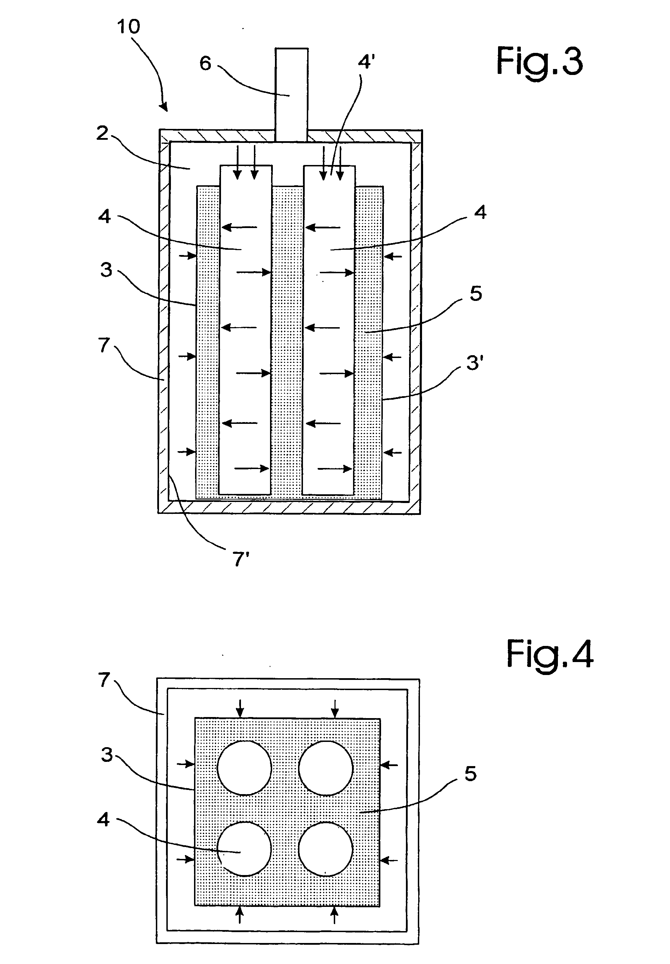

Gemäss Fig.3 und Fig.4 ist in die vom Behälter 7 gebildete dampfdicht verschliessbare Kammer 2 mindestens ein Aufnahmebehälter 3 eingesetzt, der von den Behälterwänden 7' einen Abstand hat. Dieser Aufnahmebehälter 3 besteht aus einem dampfdurchlässigen Material, beispielsweise perforiertem, rostfreiem Metall oder Kunststoff als selbsttragender Körper. Er könnte indessen auch als nicht eigensteifer Sack oder dergleichen ausgebildet sein, sofern eine Aufhängevorrichtung vorhanden ist. Ausserdem können auf ein starres oder schlaffes Gerüst Ueberzüge aus Stoff oder Kunststoff angebracht werden.

In diesen Aufnahmebehälter 3 werden mehrere, im vorliegenden Beispiel vier rohrförmige Hohlkörper 4 mit Abstand zur Behälterwand 3' und mit gegenseitigem Abstand eingesetzt, wie dies aus Fig. 2 hervorgeht. Selbstverständlich können noch mehr als vier solcher Hohlkörper 4 vorgesehen sein.

Diese rohrförmigen Hohlkörper 4 sind oben stirnseitig offen, innen leer und ihre Wandung ist dampfdurchlässig. Als Material für die Hohlkörper 4 eignen sich perforierte Metalle oder Kunststoffe, wobei auf ein selbsttragendes Rohrgerüst auch Ueberzüge aus Textilmaterial angebracht sein können. Die Querschnittsform der Hohlkörper 4 kann insbesondere rund, oval oder polygonal sein. Diese Hohlkörper 4 weisen vorzugsweise einen Aussendurchmesser von 200 bis 300 Millimetern auf und sie sind vorteilhaft mit einem Abstand zueinander von 50 bis 200 Millimetern positioniert. Sie können bis zwei Meter oder noch höher gebaut sein.

In die Zwischenräume zwischen dem Behälter 3 und den Hohlkörpern 4 und zwischen den Hohlkörpern 4 wird das zu entkeimende Gut 5 eingefüllt. Bei einer Dampfzufuhr in die Kammer 2 dringt der Dampf sowohl durch die Wandung des Aufnahmebehälters 3 als auch durch die Wandung der Hohlkörper 4 in das zu entkeimende Gut ein und bewirkt eine physikalisch definierte Entkeimung. In dem zu entkeimenden Gut 5 wird somit von mehreren Seiten vorerst ein Vakuum erzeugt und dieses dann mit Dampf durchsetzt, wie dies in Fig. 2 mit Pfeilen angedeutet ist.

Anstelle eines einzigen Aufnahmebehälters könnten in die Kammer 2 mehrere solche Aufnahmebehälter 3 eingesetzt werden. Ferner könnte statt einer stehenden Lage der Hohlkörper 4 eine liegende Lage gewählt werden. Es könnten auch mehrere Behälter neben- oder übereinander beispielsweise für Haselnüsse angeordnet sein.

Die Dampfzufuhr in die dicht geschlossene Kammer 2 der Entkeimungsanlage erfolgt durch einen nicht näher dargestellten Dampferzeuger ausserhalb der Kammer über ein allenfalls absperrbares Dampfzufuhrrohr 6. Im Prinzip könnten die Hohlkörper auch wie Lanzen in das in den Behälter eingefüllte Keimgut eingetaucht werden.

Die Erfindung ist mit dem erläuterten Ausführungsbeispiel ausreichend dargetan. Im Prinzip könnten die offenen Stirnseiten 4' der Hohlkörper 4 direkt an eine Dampfquelle angeschlossen sein und der Behälter 3 nicht noch zusätzlich durchlässig ausgebildet und von Dampf umgeben sein. Die Kammer 2 könnte aber dann auch separat an die Dampfquelle 6 angeschlossen sein.

## Patentansprüche

1. Beschickungssystem für eine Anlage zum Entkeimen vorzugsweise von Lebensmitteln, pharmazeutischen oder kosmetischen Produkten, mit einer von einem Beschickungsbehälter (27) gebildeten Kammer (22), die mehrere dampfdurchlässige, im Abstand zueinander angeordnete Hohlkörper (24) aufweist, zwischen welche das Entkeimungsgut (5) für die Entkeimung einfüllbar ist, **dadurch gekennzeichnet, dass**
in der Kammer (22) plattenförmige Hohlkörper (24) in Reihe voneinander beabstandet angeordnet sind, die in die Kammer (22) schubladenartig einschiebbar sind, so dass die Anzahl der eingesetzten Hohlkörper (24) je nach Entkeimungsgut (5) variiert werden kann.

2. Beschickungssystem nach Anspruch 1, **dadurch gekennzeichnet, dass** die vom Entkeimungsgut (5) beaufschlagten Seitenwände (34) der plattenförmigen Hohlkörper (24) aus einem dampfdurchlässigen Material hergestellt sind, beispielsweise aus einem Gewebe.

3. Beschickungssystem nach Anspruch 1 oder 2, **dadurch gekennzeichnet, dass** der in einen Autoklav (39) hineinstellbare Beschickungsbehälter (27) ebenfalls dampfdurchlässig ausgebildet ist, um Dampf in die Innenseite des Behälters einzuführen, so dass das an den Innenwänden (27') anliegende Entkeimungsgut (5) entsprechend entkeimt wird.

## Claims

1. Feeding system for an installation for sterilization preferably of food, pharmaceutical or cosmetic products, with a chamber (22) formed by a feeding container (27) comprises a plurality of vapour-permeable hollow bodies (24) interspaced and between each other the sterilization product (5) to be sterilised can be placed, **characterised in that**
in the chamber (22) plate-shaped hollow bodies (24) are arranged interspaced-in row, which are drawer like retractable in the chamber (22), so that the number of the inserted hollow bodies (24) can be varied depending on the sterilization product (5).

2. Feeding system according to claim 1, **characterised in that** the side walls (34) of the plate-shaped hollow bodies (24), pressurized by the sterilization product (5), are manufactured of a vapour-permeable material, for example of a texture.

3. Feeding system according to claim 1 or 2, **characterised in that** the feeding container (27), which can be placed in an autoclave (39), is made also vapour-permeable to implement vapour into the inside of the container, so that the sterilization product (5) adjacent to the inner walls (27') will be sterilized.

## Revendications

1. Système d'alimentation d'une installation de stérilisation avantageusement de produits alimentaires, pharmaceutiques ou cosmétiques, lequel système d'alimentation comporte une chambre (22) qui est formée par un conteneur d'alimentation (27) et qui comprend plusieurs corps creux (24), perméables à la vapeur et disposés à distance les uns des autres, entre lesquels le produit à stériliser (5) peut être introduit pour être stérilisé, **caractérisé en ce que**
des corps creux (24) en forme de plaque sont disposés en série, en étant espacé les uns des autres, dans la chambre (22) et peuvent être introduits dans la chambre (22) à la manière d'un tiroir de sorte que le nombre de corps creux insérés (24) peut varier en fonction du produit à stériliser (5).

2. Système d'alimentation selon la revendication 1, **caractérisé en ce que**, pour le produit à stériliser (5), les parois latérales (34) des corps creux en forme de plaque (24) sont fabriquées à partir d'un matériau perméable à la vapeur, par exemple à partir d'un tissu.

3. Système d'alimentation selon la revendication 1 ou 2, **caractérisé en ce que** le conteneur d'alimentation (27), insérable dans un autoclave (39), est conformé pour être également perméable à la vapeur afin d'introduire de la vapeur à l'intérieur du conteneur de sorte que le produit à stériliser (5) près des parois intérieures (27') est stérilisé de façon appropriée.
